# EUROPEAN PATENT APPLICATION

(11) **EP 4 578 472 A1**
(43) Date of publication of application: **02.07.2025**
(21) Application number: 24223366.6
(22) Date of filing: 27.12.2024
(51) Int. Cl.: A61M 1/02, A61M 1/36

(54) **AUTOMATED SEQUENTIAL INCUBATIONS DURING FLUID PROCESSING**

(30) Priority: 27.12.2023 US 202363615143 P
(71) Applicant: Fenwal, Inc., Lake Zurich, IL 60047 (US)
(72) Inventor: OLSON, Bret M., Lake Zurich, 60047 (US); WOLOK, Kyle, Lake Zurich, 60047 (US); ELICSON, Jonathan, Lake Zurich, 60047 (US); KADAKIA, Avnie A., Lake Zurich, 60047 (US)
(74) Representative: Maikowski & Ninnemann Patentanwälte Partnerschaft mbB

(57) **Abstract**

A fluid processing method and an associated system are provided. The fluid processing method includes a first and second incubation stage which are performed sequentially without any intermediate steps. The fluid processing method creates a second incubation fluid product from a biological fluid.

## Description

### TECHNICAL FIELD

The present disclosure is directed to the processing of biological fluid using a disposable fluid circuit and a reusable processing device to generate a product and a method of processing a biological fluid. More particularly, the present disclosure is directed to the processing of biological fluid with sequential incubation stages, resulting in sequential incubation products.

### BACKGROUND

The processing of biological fluid such as blood or blood components typically involves using a reusable processing device ("hardware") and a disposable fluid circuit adapted for mounting or other association with the reusable device. The fluid circuit typically includes (plastic) bags and associated tubing that defines a flow path through the circuit. The disposable fluid circuit may also include one or more separation devices where the biological fluid/cells can be separated into two or more components, washed or otherwise processed. Separation devices may separate the biological fluid based on centrifugal separation and/or, as described below, membrane separation.

Occasionally, it is desired to include an incubation stage or stages when processing a biological fluid. Current systems require separation stages between successive incubation stages or require a manual step separate from the system from an additional incubation step. However, there are certain products that require sequential incubation. The system and method described herein provides for automated sequential incubation stages in an automated fluid processing system and method.

### SUMMARY

In one aspect, a fluid processing system includes a source container, a fluid circuit connectable to the source container, at least one pump, at least one clamp, and a controller. The fluid circuit includes a separator, an in-process container, and tubing connecting the components of the fluid circuit. The controller is coupled to the at least one pump, separator, and the at least one clamp. The controller is configured to actuate the at least one pump, separator, and the at least one clamp to sequentially execute a first incubation stage and a second incubation stage within an incubation loop that includes the separator, the in-process container, and tubing which connects the in-process container and the separator.

According to another aspect, a fluid processing method includes conveying a source fluid to an in-process container, executing a first incubation stage, and executing a second incubation stage. The first incubation stage includes adding a first fluid reagent to the source fluid in the in-process container to create a first fluid product and mixing the first fluid product to generate a first incubation fluid product. The second incubation stage includes adding a second fluid reagent to the in-process container and the first incubation fluid product to create a second fluid product and mixing the second fluid product to generate a second incubation fluid product. The first and second incubation stages are executed sequentially without any intervening steps.

### BRIEF DESCRIPTION OF THE FIGURES

The disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.
Figure 1 is a frontal view of a reusable fluid processing device with a disposable fluid circuit loaded thereon.
Figure 2 is a schematic view of the control circuitry of the device of Figure 1.
Figure 3 is a schematic view of one embodiment of a disposable fluid circuit useful in the systems and methods described herein;
Figure 4 is an enlarged schematic view of one embodiment of the incubation loop of the disposable fluid circuit useful in the systems and methods described herein; and
Figure 5 is a perspective view of a separation/washing device using a spinning membrane.

### DETAILED DESCRIPTION

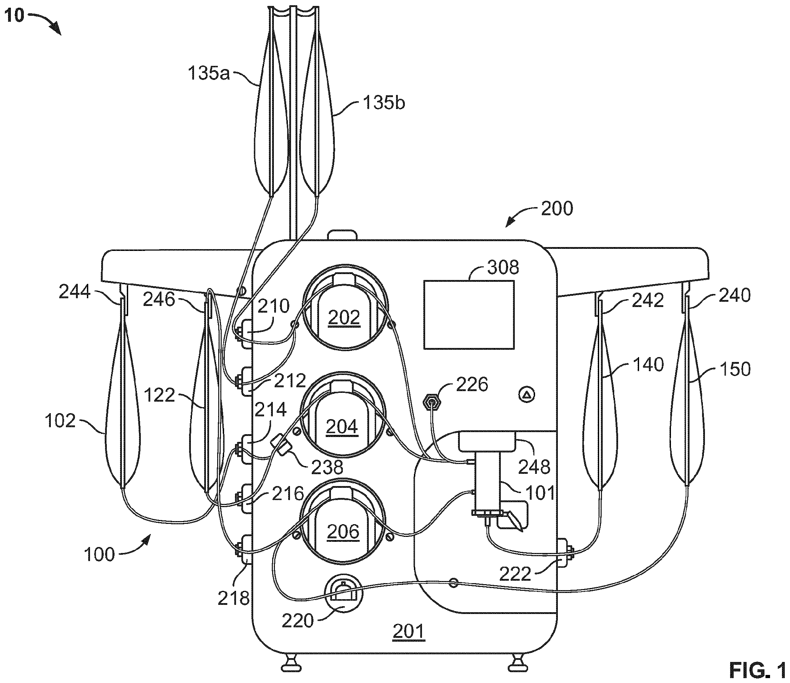

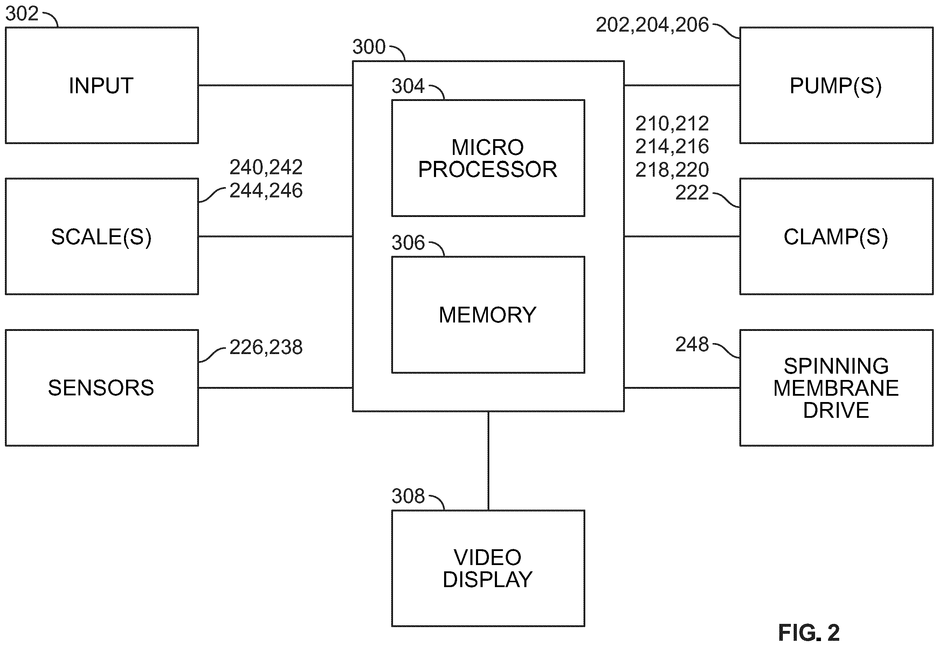

As illustrated in Figs. 1 and 2, a fluid processing system 10 includes a disposable set 100 and a device 200 to receive a biological fluid to be processed, and a control unit (or controller) 300 coupled to the device 200, the controller 300 configured to operate the system 10 according to a procedure or process to produce or generate a product that is disposed in a product container.

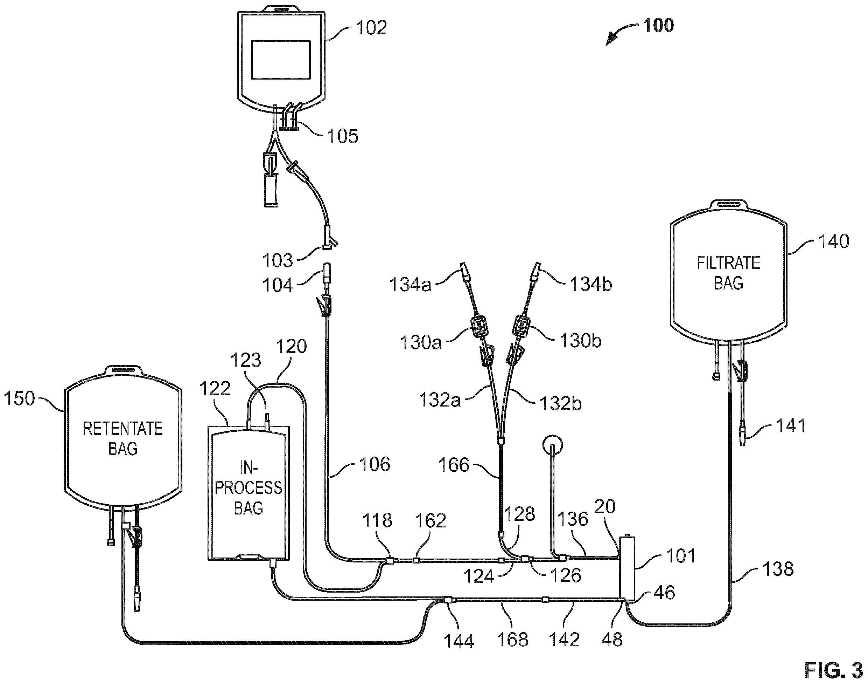

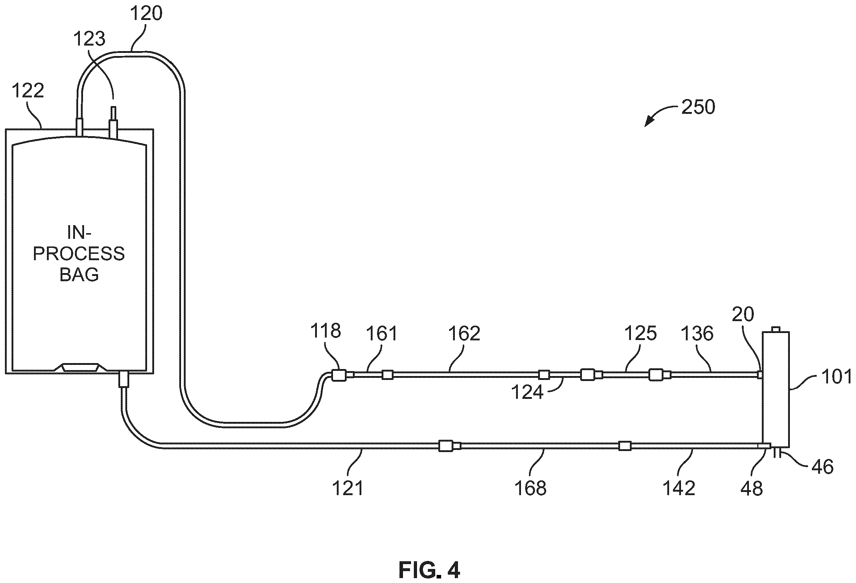

As explained in detail below, the system 10 includes a disposable processing fluid circuit 100 (see also Fig. 3, 4) and reusable device 200. According to the illustrated embodiments in Figs. 1, 3 and 4, the disposable fluid circuit 100 may include a spinning membrane 101, at least one container 102, 122, 135a, 135b, 140, 150 and tubing 106, 120, 121, 124, 125, 128, 132a, 132b, 136, 142, 161, 162, 166, 168 connecting the spinning membrane 101 and the one or more containers 102, 122, 135a, 135b, 140, 150. As is also illustrated, the device 200 may include at least one drive 248 to spin the spinning membrane 101, at least one scale 240, 242, 244, 246 to weigh the at least container 102, 122, 140, 150 and contents thereof, and at least one pump 202, 204, 206 to receive the tubing 162, 166, 168 and pump fluid through the tubing 162, 166, 168 by peristaltic action, for example, although other types of pumps and pumping action may be used. The controller 300 may, according to the embodiments, include a programmable microprocessor 304, which microprocessor 304 may be coupled to the at least one input 302 and may be programmed to operate the system according to a process.

Thus, the fluid processing systems disclosed herein typically include a reusable separation device and one or more disposable processing circuits adapted for association with the reusable device, which device and circuit(s) are operated by a controller. The reusable separation device may be any device that can provide for the automated processing of biological fluid. "Biological fluid" includes without limitation blood and blood components, and blood cells, such as red cells, white cells and platelets. By "automated," it is meant that the device can be programmed to carry out the processing steps of a biological fluid processing method without substantial operator involvement. Of course, even in the automated system of the present disclosure, it will be understood that operator activity may be involved, including the loading of the disposable fluid circuits and entering processing parameters. Additional manual steps may be required as well. However, the reusable device can process biological fluid through the disposable circuit(s) described below without substantial operator intervention.

The illustrated processing device is typically capable of effecting the separation of a biological fluid that includes biological cells into two or more components or fractions. Thus, the device may generate conditions that allow for the separation of a biological fluid into selected components or fractions. The device may also be used to perform multiple incubations.

One preferred device for separating biological fluid into its constituent components or fractions uses a spinning porous membrane. An example of such device is the Autopheresis C^{®} sold by Fenwal, Inc. of Lake Zurich, Illinois, which is an affiliate of Fresenius Kabi AG of Bad Homburg, Germany. A detailed description of a spinning membrane may be found in U.S. Patent No. 5,194,145 to Schoendorfer, which is incorporated by reference herein in its entirety, and in International (PCT) Application No. PCT/US2012/028492, filed March 9, 2012, the contents of which are also incorporated herein in their entirety. In addition, systems and methods that utilize a spinning porous membrane are also disclosed in U.S. Provisional Patent Application No. 61/537,856, filed on September 22, 2011, and International (PCT) Application No. PCT/US2012/028522, filed March 9, 2012, the contents of each are incorporated herein by reference. A further example of a cell or component processing system that utilizes a spinning membrane is the Lovo Cell Processing System which is described in US Patent Nos. 11,666,687; 10,781,001; 11,827,398; 11,191,879; 10,654,000; and 10,449,263, the contents of each are incorporated herein by reference.

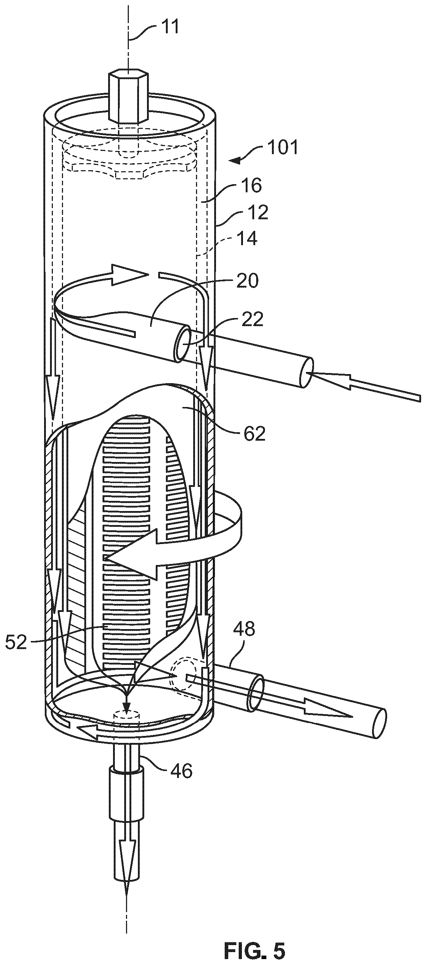

The references identified above describe a membrane-covered spinner having an interior collection system disposed within a stationary shell. While a detailed discussion of the separation device is beyond the scope of this application, the spinning membrane separation device is shown in Fig. 5 of the reference cited and is discussed below in general terms. In another embodiment, the reusable device may generate a centrifugal field to effect separation. The separator in the current disclosure is multifunctional in that it can be used to separate components of a fluid and/or it can be utilized to move fluid and effectively mix components during an incubation stage.

Turning now to Figs. 3 and 4, the systems described herein include at least one disposable fluid circuit 100 for use in the processing of biological fluid. While the circuits described herein may be used as stand-alone circuits, more preferably, at least two or more disposable fluid circuits are used in combination and in series for the separation, washing, volume reduction and/or other processing of a biological fluid. Circuit 100 may include an integrated separation device, such as, but not limited to, the spinning membrane 101 described above. Circuit 100 may also include waste container 140, product container 150, and in-process container 122. Disposable fluid circuits of the type described below may further include sampling assemblies (not shown) for collecting samples of source biological fluid, "final" product, or other intermediate products obtained during the biological fluid processing.

As will be seen in the Figures and described in detail below, the disposable fluid processing circuits include tubing that defines flow paths throughout the circuits, as well as access sites for sterile or other connection to containers of processing solutions, such as wash solutions, treating agents, or sources of biological fluid. As shown in Fig. 3, the tubing of circuit 100 includes spaced tubing segments identified by reference numerals 162, 166, 168. The tubing segments are provided for mating engagement with the peristaltic pumps 202, 204, 206 of the reusable device hardware 200 discussed below. The containers and the plastic tubing are made of conventional medical grade plastic that can be sterilized by sterilization techniques commonly used in the medical field such as, but not limited to, radiation or autoclaving. Plastic materials useful in the manufacture of containers and of the tubing in the circuits disclosed herein include plasticized poly(vinyl chloride). Other useful materials include acrylics. In addition, certain polyolefins may also be used.

As will be apparent from the disclosure herein, source containers may be attached in sterile fashion to the circuit 100. Source containers 102 for connection to one disposable circuit may be the product containers 150 of another circuit used in an earlier step of the overall method of processing. Alternatively, the contents of a product container 150 may be further processed or separated and then transferred in sterile fashion to the source container 102 of a later-in-series fluid circuit.

The biological fluid to be treated is typically provided in a source container 102, shown in Fig. 3 as (initially) not connected to the disposable set. As noted above, source container 102 may be attached (in sterile fashion) at the time of use. Source container 102 has one or more access sites 103, 105, one of which may be adapted for (sterile) connection to fluid circuit 100 at docking site 104. Preferably, source containers may be attached in a sterile manner by employing sterile docking devices, such as the BioWelder, available from Sartorius AG, or the SCD IIB Tubing Welder, available from Terumo Medical Corporation. A second access port 105 may also be provided for extracting fluid from the source container 102.

As further shown in Fig. 3, tubing segment 106 extends from docking site 104 and is connected to further downstream branched-connector 118. Branched-connector 118 communicates with tubing 106 and tubing 120, which provides a fluid flow path from "in-process" container 122 which includes at least one access site 123, described in detail below. Tubing segment 124 extends from branched-connector 118 and is joined to a port of further downstream branched-connector 126. A separate flow path defined by tubing 128 is also connected to a port of branched-connector 126.

In accordance with the fluid circuit of Fig. 3, one or more containers of wash or other processing/treating solution may be attached (or pre-attached) to set 100. As shown in Fig. 3, tubings 132a, 132b (defining a flow path) preferably include and terminate in an access site such as spike connectors 134a, 134b. Access sites 134a, 134b are provided to establish flow communication with containers 135a, 135b (shown in Fig. 1) of a wash fluid, such as saline or other solution. Tubings 132a, 132b may include in-line sterile barrier filters 130a, 130b for filtering any particulate from a fluid before it enters the flow path leading to second branched-connector 126 and, ultimately separator 101 or in-process container 122. In one embodiment, the sterile barrier filters 130a, 130b may be 0.2 µm filters. The wash medium or fluid flows from the wash fluid source through tubing segments 132a, 132b where it is filtered by the sterile barrier filters 130a, 130b described above, and then passes through tubing 128 to the input of the branched-connector 126 described above.

Tubing segment 136 defines a flow path connected at one end to branched-connector 126 and to an inlet port 20 of the separator 101 or in-process container 122. Preferably, in accordance with the present disclosure, separation device 101 is a spinning membrane separator of the type described in U.S. Patent No. 5,194,145 and U.S. Patent No. 5,053,121, which are incorporated by reference, U.S. Provisional Patent Application Serial No. 61/451,903 and PCT/US2012/028522, also previously incorporated herein by reference.

As shown in Fig. 3 (and described in detail in connection with Fig. 5), the spinning membrane separator 101 has at least two outlet ports. Outlet 46 of separator 101 receives the waste from the wash (i.e., the diluted suspension medium) and is connected to tubing 138, which defines a flow path to waste product container 140. The waste product container 140 includes a further connection port 141 for sampling or withdrawing the waste from within the product container.

Separation device 101 preferably includes a second outlet 48 that is connected to tubing segment 142 for directing the desired biological cell/fluid product to the in-process container(s) 122 or the product container 150. To permit this, the other end of tubing segment 142 is connected to branched-connector 144, which branches into and defines a flow path to one or more in-process containers 122 and a flow path to a "final" product container 150. The product container 150 may also include a sampling assembly (not shown).

Shown in greater detail in Fig. 4, is the incubation loop 250 of the fluid circuit, which includes components of the described fluid circuit. The incubation loop includes the in-process bag 122, the separator 101, and the tubing line segments 120, 121, 161, 162, 124, 125, 136, 142, 168 connecting the components. For each incubation, the components of this incubation loop are utilized. The biological fluid passes from the in-process bag 122 through line segments 120, 161, 162, 124, 125 and 136 to the separator 101 and back to the in-process bag 122 through line segments 142, 168, and 121.

Device 200 may be of compact size suitable for placement on a tabletop of a lab bench and adapted for easy transport. Alternatively, device 200 may be supported by a pedestal that can be wheeled to its desired location. In any event, device 200 includes a plurality of peristaltic pumps such as pumps 202, 204 and 206 on front panel 201. Pump segments of the disposable fluid circuit (described above) are selectively associated with peristaltic pumps 202, 204, and 206. The peristaltic pumps articulate with the fluid set of Fig. 3 at the pump segments identified by reference numerals 162, 166, 168 and advance the fluid within the disposable set, as will be understood by those of skill in the art. Device 200 also includes clamps 210, 212, 214, 216, 218, 220 and 222. The clamps are used to control the flow of the cell suspension through different segments of the disposable set, as described above.

Device 200 may also include several sensors to measure various conditions. The output of the sensors is utilized by device 200 to operate one or more wash or processing cycles. One or more pressure transducer sensor(s) 226 may be provided on device 200 and may be associated with a disposable set 100 at certain points to monitor the pressure during a procedure. Pressure transducer 226 may be integrated into an in-line pressure monitoring site (at, for example, tubing segment 136), to monitor pressure inside separator 101. Air detector sensor 238 may also be associated with the disposable set 100, as necessary. Air detector 238 is optional and may be provided to detect the location of fluid/air interfaces.

Device 200 may also include weight scales 240, 242, 244, and 246 from which the final product container 150, waste container 140, the source container 102 and the in-process container 122, respectively, may depend and be weighed. The weights of the bags are monitored by weight sensors and recorded during a washing or other procedure. From measurements of the weight sensors, the device determines whether each container is empty, partially full, or full and controls the components of device 200, such as the peristaltic pumps 202, 204 and 206 and clamps 210, 212, 214, 216, 218, 220 and 222.

Device 200 includes at least one drive unit or "spinner" 248, which causes the indirect driving of the spinning membrane separator 101. Spinner 248 may consist of a drive motor connected and operated by device 200, coupled to turn an annular magnetic drive member including at least a pair of permanent magnets. As the annular drive member is rotated, magnetic attraction between corresponding magnets within the housing of the spinning membrane separator causes the spinner within the housing of the spinning membrane separator to rotate.

Turning to Fig. 5, a spinning membrane separation device, generally designated 101, is shown. Such a device 101 forms part of the disposable circuit 100.

Device 101 includes a generally cylindrical housing 12, mounted concentrically about a longitudinal vertical central axis. An internal member 14 is mounted concentric with the central axis 11. Housing 12 and internal member 14 are relatively rotatable. In the preferred embodiment, as illustrated, housing 12 is stationary and internal member 14 is a rotating spinner that is rotatable concentrically within cylindrical housing 12, as shown by the thick arrow in Fig. 5. The boundaries of the flow path are generally defined by gap 16 between the interior surface of housing 12 and the exterior surface of rotary spinner 14. The spacing between the housing and the spinner is sometimes referred to as the shear gap. The shear gap may be approximately 0.02 - 0.06 inches (0.05 - 0.15 cm) and may be of a uniform dimension along axis 11, for example, where the axis of the spinner and housing are coincident. The shear gap may also vary circumferentially for example, where the axis of the housing and spinner are offset.

The shear gap also may vary along the axial direction, for example preferably an increasing gap width in the direction. Such a gap width may range from about 0.02 to about 0.075 inches (0.05 - 0.19 cm). The gap width could be varied by varying the outer diameter of the rotor and/or the inner diameter of the facing housing surface. The gap width could change linearly or stepwise or in some other manner as may be desired. In any event, the width dimension of the gap is preferably selected so that at the desired relative rotational speed, Taylor-Couette flow, such as Taylor vortices, are created in the gap.

With reference to Fig. 5, biological fluid is fed from an inlet conduit 20 through an inlet orifice 22, which directs the fluid into the fluid flow entrance region in a path tangential to the circumference about the upper end of the spinner 14. At the bottom end of the cylindrical housing 12, the housing inner wall includes an exit orifice 48.

Cylindrical housing 12 is completed by a bottom end housing terminating in an outlet orifice 46 concentric with the central axis. The outlet orifice 46 may also include a cap, clamp, or other closure mechanism, which can be utilized to close the orifice 46 during an incubation step in which nothing is exiting the separator at this outlet. In one embodiment, the instrument clamp 222 on the tubing line 138 is used to stop the fluid flow.

In the illustrated embodiment, the surface of the rotary spinner 14 is at least partially, and is preferably substantially or entirely, covered by a cylindrical porous membrane 62. The membrane 62 may have a nominal pore size between 0.8 and 10 microns (µm), for example. Membranes may be fibrous mesh membranes, cast membranes, track-etched membranes or other types of membranes that will be known to those of skill in the art. For example, in one embodiment, the membrane may have a polyester mesh (substrate) with nylon particles solidified thereon, thereby creating a tortuous path through which only certain sized components will pass. In an embodiment, the nylon membrane may have a pore size of approximately 0.8 µm and a thickness of approximately 150 µm or greater. Membranes of this type will typically retain all cellular components (e.g., red blood cells, white blood cells) and certain formed blood components, e.g., platelets. In another embodiment, the membrane may be made of a thin (approximately 10 µm thick) sheet of unsupported polycarbonate, for example, with a pore size of approximately 4.0 µm. In this embodiment, pores (holes) may be cylindrical and larger than those described above. The pores may be sized to allow small formed components (e.g., platelets, microparticles, etc.) to pass, while the desired cells (e.g., white blood cells and larger red blood cells) are collected.

Having thus described the system, including disposable circuit 100 and reusable device 200, reference is made to Fig. 2 to discuss additional details of the control unit or controller 300, which is also part of the system 10. As mentioned above, the controller 300 may include a microprocessor 304 (which, in fact may include multiple physical and/or virtual processors). According to other embodiments, the controller 300 may include one or more electrical circuits designed to carry out the actions described herein. In fact, the controller 300 may include a microprocessor and other circuits or circuitry. In addition, the controller 300 may include one or more memories 306. The instructions by which the microprocessor 304 is programmed may be stored on the memory 306 associated with the microprocessor 304, which memory/memories 306 may include one or more tangible non-transitory computer readable memories, having computer executable instructions stored thereon, which when executed by the microprocessor 304, may cause the microprocessors 304 to carry out one or more actions as described below.

As is also illustrated in Fig. 2, the controller 300 may be coupled to one or more of the structures described above, for example to receive information (e.g., in the form of signals) from these structures or to provide commands (e.g., in the form of signals) to these structures to control the operation of the structures. As illustrated, the controller 300 may be coupled to the scales 240, 242, 244, 246, the sensors 226, 238 and the at least one input 302 to receive information from those devices. Additionally, the controller 300 may be coupled to the pumps 202, 204, 206, the clamps 210, 212, 214, 216, 218, 220, 222, and the drive 248 to provide commands to those devices to control their operation. It may also be possible that the controller 300 receives information from and provides commands to a given structure, such as one of the structures already mentioned. The controller 300 may be directly electrically connected to these structures to be coupled to them, or the controller 300 may be directly connected to other intermediate equipment that is directly connected to these structures to be coupled to them.

The at least one input 302 may include a number of different devices according to the embodiments described herein. For example, the input 302 could include a keyboard or keypad by which a user may provide information and/or instructions to the controller 300. Alternatively, the input 302 may be a touch screen, such as may be used in conjunction with a video display 308 that is disposed on the front panel 201 of the device 200, the video display 308 also being coupled to the controller 300. The input could also include a reader or scanner, such as a barcode reader or scanner or an RFID reader. The assembly of the input/touch screen 302 and video display 308 may be one of the afore-mentioned structures to which the controller 300 is coupled from which the controller 300 receives information and to which the controller 300 provides commands. According to still other embodiments, the input 302 may be in the form of computer equipment that permits the fluid processing system including the controller 300 to communicate (whether via wires, cables, etc. or wirelessly) with other fluid processing systems over a local network, or with other fluid processing systems or other computer equipment (e.g., a server) over local networks, wide area networks, or the Internet. According to such an embodiment, the input may include an internal transmitter/receiver device.

Having discussed the structure of embodiments of the fluid processing system disclosed herein, the operation of the fluid processing system is now discussed. The fluid processing method steps of the current disclosure may be performed alone or within a larger fluid processing method.

In general, the operator may first activate (e.g., switch on) the device 200, at which point the device 200 conducts self-calibration checks, including the checking of the peristaltic pumps 202, 204, 206, clamps 210, 212, 214, 216, 218, 220, 222, and sensors 226, 238. Device 200 may then prompt the user to enter or modify process parameters using the input 302, including by way of example and not by way of limitation: number of incubations within each cycle, incubation volume, incubation wash media, incubation duration, incubation flow rate, incubation spinner revolution rate, pre- and post-incubation pauses, and pre- and post-incubation pause text. The number of incubations can vary from 0 (which is disabled), 1 (single) or 2 (double/sequential). The incubation volume can vary from 5-800 mL, and can include the ability to target the minimum achievable incubation volume based on all procedure inputs and estimations. The specific incubation wash media can be selected, such as, solution 1 or solution 2, which is the user provided solution used by the system to pump the cell suspension to the incubation volume, these solutions being attached at 123. The incubation can be varied from 1-99 minutes. The incubation flow rate, or flow rate through the incubation loop, can vary from 0-200mL/min, wherein 0 indicates no flow and off-line agitation via a rocker tray. The incubation spinner revolution rate can be between 0-4500 RPM (0 if not being used to circulate or mix the incubation solution). There can be pre- or post-incubation pauses and during these pauses the text on the screen may indicate instructions for the user to follow. The device 200 may then prompt the operator to mount the disposable set 100, after which device 200 automatically checks to determine whether the disposable set 100 is properly installed. Once the set 100 is properly installed, the controller 300 prompts the operator to connect the source of biological fluid (e.g., 102 of Fig. 3) via a spike connector or sterile connection (e.g., 103, 104 of Fig. 3) and any wash medium (e.g., 135a, 135b of Fig. 3) via a spike connector (e.g., 134a, 134b of Fig. 3).

Once the operator confirms that the solutions are connected, the controller 300 primes the disposable set 100. In the embodiment discussed above, the set 100 may be primed with saline, although other biocompatible aqueous solutions may also be used. The controller then commences the biological fluid processing. The controller may direct the source fluid directly to the in-process container or perform a separation before. If a separation is done, the controller 300 then initiates the transferring from source container (e.g., 102 of Fig. 3) through the set to the spinning membrane separator 101 via the operation of one or more peristaltic pumps 202, 204 and 206. In a similar fashion, the wash medium is delivered from its container (e.g., 135a, 135b of Fig. 3) through the set to the spinning membrane separator 101. The biological cells are then collected in an in-process bag (e.g., 122 of Fig. 3) while supernatant is separated and removed to waste container (e.g., 140 of Fig. 3).

If an initial separation is not desired, the biological source material may be transferred to the in-process container 122.

Once biological cells are collected in the in-process bag 122, either from a source container or as a separation product, a reagent or solution may be added to the biological cells in the in-process bag to initiate a first incubation stage. This can be done at port 123 of the in-process bag, such as by syringe or other transfer device. The reagent or solution can be any known reagent or solution depending on the incubation process desired. The reagents can be reagents containing antibody complexes that allow for specific labeling of desired cells in suspension. For example, the reagent may include antibody complexes recognizing CD4+ cells and magnetic particles. Once the target cells (CD4+) are labeled they can be removed from the remaining cell suspension with a magnet. The combination of the reagent or solution and the biological cells of the source fluid produce a first fluid product.

This first fluid product is then mixed or agitated to generate a first incubation fluid product. There may be a waiting period before this agitation or mixing is started. The waiting period can be 15 min, 30 min, 1 hr, or any amount of time chosen by the user/operator. The waiting period can also be 0 minutes, or the mixing is initiated immediately after the introduction of the reagent or solution. This mixing can be accomplished by circulating the first fluid product from the in-process bag 122 to the separator and eventually back to the in-process bag 122 or can be done by agitating with an external device offline, such as a rocker tray or by manual mixing where the user inverts the bag repeatedly. The mixing step can be done for a set amount of time, such as between 1 and 99 minutes or 10-75 mins, or 25-50 minutes. The set amount of time can be any integer between 1-99. The flow of the liquid through the separator and back to the in-process bag can also be regulated to a certain speed, such as between 0 mL/min and 200 mL/min, the speed can be between 50 mL/min and 150 mL/min or between 75 mL/min and 125 mL/min. The flow rate can be any integer between 0 and 200. Once the first incubation fluid product is adequately mixed, the first incubation stage ends and the first incubation fluid product is in the in-process bag 122.

Immediately after the first incubation stage a second incubation stage is executed. The first incubation fluid product is in the in-process bag 122 of the fluid circuit. A second fluid reagent or solution is added to the first incubation fluid product at the port 123 of the in-process bag 122. This can be done at port 123 of the in-process bag 122, such as by syringe or other transfer device. The reagent or solution can be any known reagent or solution depending on the incubation process desired. The combination of the reagent or solution and the first incubation fluid product of the source fluid produces a second fluid product.

This second fluid product is then mixed or agitated to generate a second incubation fluid product. There may be a waiting period before this agitation or mixing is started. The waiting period can be 15 min, 30 min, 1 hr, or any amount of time chosen by the user/operator. The waiting period can also be 0 minutes, or the mixing is initiated immediately after the introduction of the reagent or solution. This mixing can be accomplished by circulating the first fluid product from the in-process bag 122 to the separator and eventually back to the in-process bag 122 or can be done by agitating with an external device offline, such as a rocker tray. The mixing step can be done for a set amount of time, such as between 1 and 99 minutes or 10-75 mins, or 25-50 minutes. The set amount of time can be any integer between 1-99. The flow of the liquid through the separator and back to the in-process bag can also be regulated to a certain speed, such as between 0 mL/min and 200 mL/min, the speed can be between 50 mL/min and 150 mL/min or between 75 mL/min and 125 mL/min. The flow rate can be any integer between 0 and 200. Once the second incubation fluid product is adequately mixed, the first incubation stage ends and the second incubation fluid product is in the in-process bag 122.

The first and second incubation stages are executed sequentially and without any intervening steps.

After the second incubation stage, a separation stage may be performed, the resulting second incubation fluid product may be washed, or the in-process bag may be removed with the second incubation fluid product.

If a separation stage is executed, the second incubation fluid product is transferred from the in-process bag 122 through the set to the spinning membrane separator 101 via the operation of one or more peristaltic pumps 202, 204 and 206. In a similar fashion, the wash medium is delivered from its container (e.g., 135a, 135b of Fig. 3) through the set to the spinning membrane separator 101. The biological cells are collected in either an in-process bag (e.g., 122 of Fig. 3) for additional processing or in a product container (e.g., 150 of Fig. 3), while supernatant is separated and removed to waste container (e.g., 140 of Fig. 3). An additional incubation stage may be performed either after the second incubation stage or after a separation or washing stage.

Once the processing is completed, the controller prompts the operator to sample, seal and remove the product container 150. The resulting product is a washed, concentrated, and purified cell suspension.

The systems and methods described herein may be effective, for example, in cell enrichment processing routines. These processing routines include a separation stage and at least two incubation stages.

### EXAMPLE

The fluid processing system may support enhanced CD34+ cell enrichment processing routines. Specifically, to support the enrichment of CD34+ cell populations within peripheral blood mononuclear cell (PBMC) apheresis products. The system described above will first execute a separation process to remove platelets which could negatively impact binding an antibody conjugate. The system then incubates the platelet-depleted cell suspension with an Immunoglobulin G (IgG) solution to reduce the incidence of non-specific binding of an antibody conjugate [new functionality]. The system then incubates the platelet-depleted, IgG-incubated cell suspension with an antibody conjugate. The resulting product is a washed, concentrated, and purified cell suspension.

While the foregoing discussion references embodiments in the form of a fluid processing system, other systems may incorporate this technology as well. These systems may share the technical challenges faced by the aforementioned fluid processing system, and incorporation of the technology may provide similar advantages. For example, a separation system, more particularly a filtration system, or even more particularly a microfiltration system, also may include a processor to receive a fluid to be processed and a controller. Further, certain embodiments of such a processor may include a disposable fluid circuit (which circuit may include a membrane used for filtration) and reusable hardware, and the controller may be configured to operate the processor.

Thus, an improved method and system have been disclosed for the sequential incubating and processing of a fluid. The description provided above is intended for illustrative purposes only and is not intended to limit the scope of the invention to any specific method, system, or apparatus, or device described herein except as may be explicitly delineated above.

### Aspects

Aspect 1. A fluid processing system comprising a source container, a fluid circuit connectable to the source container comprising a separator, an in-process container, tubing connecting the components of the fluid circuit, at least one pump, at least one clamp, and a controller coupled to the at least one pump, separator, and the at least one clamp and configured to actuate the at least one pump, separator, and the at least one clamp to sequentially execute a first incubation stage and a second incubation stage within an incubation loop that includes the separator, the in-process container, and tubing which connects the in-process container and the separator.

Aspect 2. The fluid processing system of Aspect 1, wherein the system further includes a display screen which allows a user to set the incubation parameters for the first and second incubation stages.

Aspect 3. The fluid processing system of Aspect 2, wherein the incubation parameters include at least one of incubation volume, incubation duration, incubation flow rate, and incubation spinner revolution rate.

Aspect 4. The fluid processing system of Aspect 1, wherein the separator is a spinning membrane separator.

Aspect 5. The fluid processing system of any of the preceding Aspects, wherein the fluid is blood, a blood component, or a mixture of blood components.

Aspect 6. The fluid processing system of any of the preceding Aspects, wherein the source container is a bag.

Aspect 7. The fluid processing system of any of the preceding Aspects, wherein the in-process container is a bag.

Aspect 8. The fluid processing system of any of the preceding Aspects, wherein the system further includes a wash container, and the fluid circuit is connectable to the wash container.

Aspect 9. The fluid processing system of any of the preceding Aspects, wherein the in-process container includes a port for adding reagent or solution.

Aspect 10. A fluid processing method comprising conveying a source fluid to an in-process container, executing a first incubation stage comprising adding a first fluid reagent to the source fluid in the in-process container to create a first fluid product and mixing the first fluid product to generate a first incubation fluid product, executing a second incubation stage comprising adding a second fluid reagent to the in-process container and the first incubation fluid product to create a second fluid product and mixing the second fluid product to generate a second incubation fluid product, wherein the first and second incubation stages are executed sequentially without any intervening steps.

Aspect 11. The fluid processing method of Aspect 10, wherein the mixing of the first incubation stage includes circulating the first fluid product and first fluid reagent through an in-process container and a separator.

Aspect 12. The fluid processing method of any of Aspects 10 and 11, wherein the mixing of the second incubation stage includes circulating the first incubation fluid product and the second fluid reagent.

Aspect 13. The fluid processing method of any of Aspects 10-12, wherein the mixing of the first incubation stage includes off-line agitation with a rocker tray.

Aspect 14. The fluid processing method of any of Aspects 10-13, wherein the mixing of the second incubation stage includes off-line agitation with a rocker tray.

Aspect 15. The fluid processing method of any of Aspects 10-14, where the method includes a separation stage before the first incubation stage.

Aspect 16. The fluid processing method of any of Aspects 10-15, wherein the first incubation stage is for a set amount of time.

Aspect 17. The fluid processing method of any of Aspects 11-16, wherein the second incubation stage is for a set amount of time.

Aspect 18. The fluid processing method of any of Aspects 10-17, wherein the first incubation stage further includes adding at least one wash media to the in-process container.

Aspect 19. The fluid processing method of any of Aspects 10-18, wherein the second incubation stage further includes adding at least one wash media to the in-process container.

## Claims

1. A fluid processing system comprising:
a source container;
a fluid circuit connectable to the source container comprising:
a separator;
an in-process container;
tubing connecting the components of the fluid circuit;
at least one pump;
at least one clamp; and
a controller coupled to the at least one pump, separator, and the at least one clamp and configured to actuate the at least one pump, separator, and the at least one clamp to sequentially execute a first incubation stage and a second incubation stage within an incubation loop that includes the separator, the in-process container, and tubing which connects the in-process container and the separator.

2. The fluid processing system of claim 1, wherein the system further includes a display screen which allows a user to set the incubation parameters for the first and second incubation stages, preferably, wherein the incubation parameters include at least one of incubation volume, incubation duration, incubation flow rate, and incubation spinner revolution rate.

3. The fluid processing system of claim 1, wherein the separator is a spinning membrane separator.

4. The fluid processing system of any of the preceding claims, wherein the fluid is blood, a blood component, or a mixture of blood components.

5. The fluid processing system of any of the preceding claims, wherein the source container is a bag.

6. The fluid processing system of any of the preceding claims, wherein the ,in-process container is a bag.

7. The fluid processing system of any of the preceding claims, wherein the system further includes a wash container, and the fluid circuit is connectable to the wash container.

8. The fluid processing system of any of the preceding claims, wherein the in-process container includes a port for adding reagent or solution.

9. A fluid processing method comprising:
conveying a source fluid to an in-process container;
executing a first incubation stage comprising:
adding a first fluid reagent to the source fluid in the in-process container to create a first fluid product; and
mixing the first fluid product to generate a first incubation fluid product;
executing a second incubation stage comprising:
adding a second fluid reagent to the in-process container and the first incubation fluid product to create a second fluid product; and
mixing the second fluid product to generate a second incubation fluid product;
wherein the first and second incubation stages are executed sequentially without any intervening steps.

10. The fluid processing method of claim 9, wherein the mixing of the first incubation stage includes circulating the first fluid product and first fluid reagent through an in-process container and a separator.

11. The fluid processing method of any of claims 9 and 10, wherein the mixing of the second incubation stage includes circulating the first incubation fluid product and the second fluid reagent.

12. The fluid processing method of any of claims 9-11, wherein the mixing of the first incubation stage and/or the second incubation stage includes off-line agitation with a rocker tray.

13. The fluid processing method of any of claims 9-12, where the method includes a separation stage before the first incubation stage.

14. The fluid processing method of any of claims 9-13, wherein the first incubation stage and/or the second incubation stage is for a set amount of time.

15. The fluid processing method of any of claims 9-14, wherein the first incubation stage and/or second incubation stage further includes adding at least one wash media to the in-process container.
